# EUROPÄISCHE PATENTSCHRIFT

(11) **EP 0 614 646 B1**
(45) Veröffentlichungstag und Bekanntmachung des Hinweises auf die Patenterteilung: **29.09.1999**
(21) Anmeldenummer: 94103398.7
(22) Anmeldetag: 07.03.1994
(51) Int. Cl.: A61B 17/02, A61B 17/34

(54) **Chirurgische Trokarhülsen**
Surgical trocar sleeve
Manchon de trocart chirurgical

(30) Priorität: 08.03.1993 DE 4307228
(43) Veröffentlichungstag der Anmeldung: 14.09.1994
(73) Patentinhaber: Tomic, Dobrivoje, Dr., D-81549 München (DE)
(72) Erfinder: Tomic, Dobrivoje, Dr., D-81549 München (DE)

(56) Entgegenhaltungen:
- EP-A- 0 515 220
- WO-A-92/08513
- WO-A-92/18056
- WO-A-92/21292
- DE-C- 3 504 292
- DE-U- 9 102 759

## Beschreibung

Die Erfindung betrifft eine Trokarhülse gemäß dem Oberbegrif des Anspruchs 1.

In der Chirurgie und insbesondere bei der intrakorporalen endoskopischen Chirurgie ist eine Trokarhülse aus EP-A-0 515 220 beckannt, jedoch ohne Möglichgkeit und Vorrichtung zum gleichzeitigen Verdrängen, Halten oder Heben der Organe, Gewebe oder Bauchdecke. Besonders grosse Nachteile zeigen die bekannten Trokarhülsen in der endoskopischen Thoraxchirurgie, in der das Fernhalten des umliegenden Gewebes/Organteile dringend erforderlich ist, so daß ein zusätzliches Instrument bzw. Halter notwendig wird. Einen grossen Nachteil weisen auch die Trokarhülsen in der laparoskopischen Chirurgie, weil sie über keine Vorrichtung zum Halten und/oder Heben der Bauchdecke verfügen.

Der Erfindung liegt die Aufgabe zugrunde, chirurgische Trokarhülsen mit beweglichen, schwenkbaren Teilen und einer Bewegungsvorrichtung zur seitlichen Schwenkung, Spreizung, Öffnung von einer oder mehreren verbundenen Plättchen, Stangen, Segmente anzugeben, zur Benützung in der Chirurgie, insbesondere bei minimal invasiven und intralorporalen endoskopischen chirurgischen Eingriffen als multifunktionellen Trokarhülsen, durch welche Endoskope, Instrumente und ähnliches in das Körperinnere eingeführt werden, und Organe, Gewebe oder die Bauchdecke verdrängt, gehalten oder gehoben werden können. Gelöst wird diese Aufgabe mit den Merkmalen, die im Kennzeichen des Einspruchs 1. definiert sind.

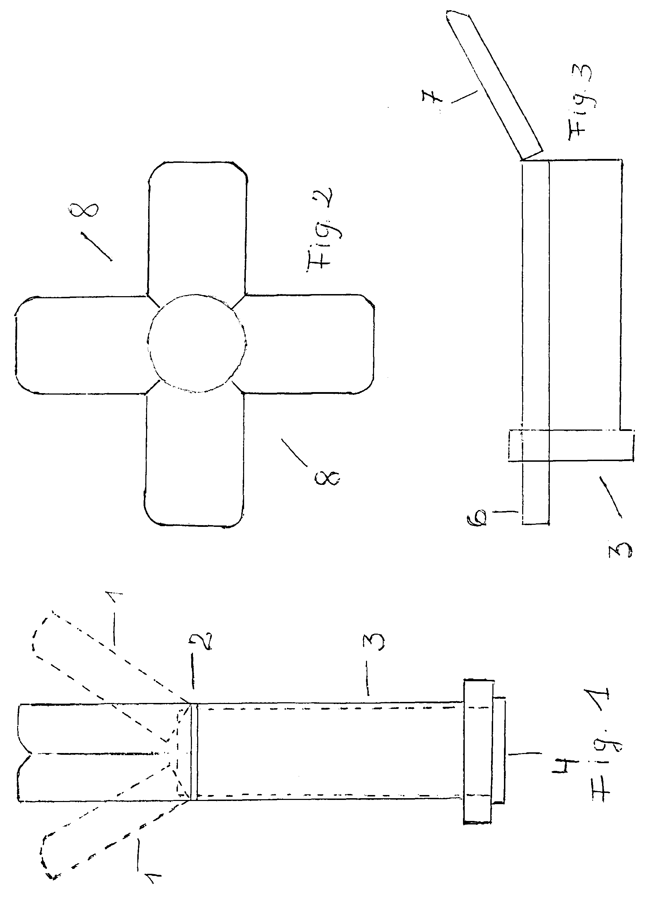

Bevorzugte Ausführungsbeispil ist es die Trokarhülse in verschiedenen Formen wie oval, rund, eliptisch und die Schaffung von Trokarhülsen mit beweglichen, schwenkbaren Teilen (Fig.1), bestehend aus einer äusseren Trokarhülse welche in ihrem distalen Teil ein oder mehrere nach aussen schwenkbaren Plättchen, Stangen, Segmenten aufweist und einer starren zweiten inneren Hülse, welche in die erste Trokarhülse eingeführt wird um die beweglichen Teile, Plättchen, Segmente zu bewegen, auszuschwenken, zu spreizen und diese in Position zu halten (Fig.2) und Trokarhülsen mit einer Vorrichtung bestehend aus einem oder mehreren Plättchen, Stangen, Segmenten oder ähnlichem welche an/oder in die Trokarhülse angebracht sind (Fig.3) und bei Betätigung bzw. Schieben dieser beweglichen Teile eine laterale Schwenkung am distalen Ende der Trokarhülse bewirkt, und diese Schwenkung/Spreizung das Organ oder Gewebe aus dem Operationsfeld hebt oder die Bauchdecke eleviert.

Die erfindungsgemäßen Trokarhülsen weisen zwei ineinander gehende Hülsen auf, welche die äussere Trokarhülse in ihrem distalen Teil eine Vorrichtung mit beweglichen schwenkbaren Teilen wie Plättchen, Segmenten, Stangen aufweist. Die beweglichen Teile sind mit dem äusseren oder inneren Hülsenteil scharnierartig verbunden. Die äussere und die innere Hülse sowie die beweglichen Teile können verschiedene Größen und Formen aufweisen, aus verschiedenen Materialien, starr, elastisch, überzogen, isoliert und/oder auswechselbar sein. Bei intrakorporalen operativen Eingriffen wird auf übliche Weise zuerst die erfindungsgemäße äussere Trokarhülse eingeführt und Trokar entfernt. Darauf wird die zweite Hülse in die erste Trokarhülse eingeführt/eingeschoben, wobei mit ihrer distalen Kante/Spitze ein oder mehrere oder alle beweglichen Teile, Segmente, Plättchen lateral nach aussen gedrückt, bewegt und je nach Plättchenform bis zu 90° geschwenkt werden. Dabei kommt es zu Verdrängen, Heben oder Fernhalten von Organen und Gewebe und der Freihaltung des Operationsfeldes, womit ein Eingriff bei guter und freier Sicht ermöglicht wird. Nach beendigung der Operation wird nach der Instrumentenentfernung zuerst die innere Hülse herausgenohmen, wobei dann alle zufor geschwenkten Teile, Plättchen, Segmente in ihre ursprungliche Ruhestellung zurückfallen, so dass dann auch die äussere Trokarhülse problemlos herausgenohmen werden kann.

Die Trokarhülsen mit erfindungsgemäßer beweglicher Schwenkungs/Spreizungsvorrichtung können bei laparoskopischen Operationen auch als Bauchdeckenhalter oder Heber fungieren, insbesondere bei Zusammenbruch des Pneumoperitoneum, bei Druckabfall von Gas/CO2 wobei auch mehrere erfindungsgemäße Trokarhülsen extern über der Bauchdecke untereinander verbunden sein können um die Bauchdecke jederzeit während des OP-eingriffes zu heben und/oder hochzuhalten bei einem Eingriff ohne Anwendung von Gasdruck.

Zum Bewegen, Offnen/Schließen und Betätigen der erfindungsgemäßen beweglichen Teile, Segmente, Plättchen können verschiedene und bekannte Vorrichtungen benützt werden wie z.B. Betätigung mittels Gewinde-Schraubendrehung. Die erfindungsgemäße Trokarhülse eignet sich besonders vorteilhaft für die Thoraxchirurgie. Die Betätigungsvorrichtung und die beweglichen Teile können auch ganz oder teilweise abnehmbar/austauschbar sein.

Die Vorteile dieser erfindungsgemäßen Trokarhülsen sind überzeugend und vielfältig. Bei Benützung/Anwendung der neuen Trokarhülse insbesondere bei bisher benötigter Zusatzinstrumente im Thorax als Lunge-Gewebehalter nicht mehr nötig. Das ist ein wesentlicher Fortschritt in der OP-Technik, wobei dem Patienten eine zusätzliche Stichwunde erspart wird und der OP-Ablauf vereinfacht und Verkürzt wird. Auch die Kostensenkung ist beachtlich gross. Die erfindungsgemäßen Trokarhülsen sind im Gegensatz zu bisher bekannten Trokarhülsen multifunktionelle Instrumente, welche sowohl zur Instrumenteneinführung als auch zum gleichzeitigen Verdrängen,Halten, Heben von Organen, Gewebe und/oder Körperteilen in der minimalinvasiven intrakorporalen Chirurgie Anwendung finden.

Die beweglichen Plättchen, Segmente oder ähnliches können ihre Grösse und/oder Form durch eine teleskopartige Vorrichtung und Betätigung verändern und abnehmbar sein.

Die folgende Zeichnung soll als Beispiel die Erfindung und Ansprüche näher erläutern.

## Patentansprüche

1. Chirurgische Trokarhülse mit beweglichen, schwenkbaren Teilen (1) und einer Bewegungsvorrichtung ausgestattet ist, die seitliche Schwenkung, Spreizung, Öffnung von damit verbundenen ein oder mehreren Plättchen, Stangen, Segmenten (2) bewirken, zur Benützung in der Chirurgie, insbesondere bei minimal invasiven und intrakorporalen endoskopischen chirurgischen Eingriffen als multifunktionelle Trokarhülse, durch welche Endoskope, Instrumente und ähnliches in das Körperinnere eingeführt werden können, und Organe, Gewebe oder die Bauchdecke verdrängt, gehalten oder gehoben werden können,
-- die chirurgische Trokarhülse aus einer äusseren (3) und einer inneren (4) Hülse besteht, wobei an den distalen Teil der äusseren ( ) oder inneren (4) Trokarhülse die Plättchen, Stangen, Segmenten (2) verbunden sind,
dadurch gekenzeichnet, dass
-- die äussere (3) und innere (4) Hülse unabhänging voneinander und frei ineinander beweglich sind, dass
-- die Plättchen, Stangen,Segmente (2) unabhängige Teile sind, die mit der äusseren (3) oder inneren (4) Trokarhülse scharnierartig verbunden sind, und dass
-- die Bewegungseinrichtung darin besteht, daß durch Relativbewegung zwischen der äusseren (3) und innere (4) Trokarhülse eine laterale Schwenkung bis zu 90° der Plättchen, Stangen, Segmente (2) am distalen Ende der Trokarhülse bewirkt.

## Claims

1. A surgical trocar sleeve with moving, swivelling parts (1) and provided with a mover which causes the lateral swivelling, spreading apart or opening of one or more small plates, rods or segments (2) connected to it, for use in surgery, in particular during minimally invasive and intracorporeal endoscopic surgical operations as a multifunctional trocar sleeve through which endoscopes, instruments and similar may be introduced into the interior of the body, and with which organs, tissues or the abdomen wall may be displaced, held back or lifted,
- the surgical trocar sleeve consists of an outer (3) and an inner (4) sleeve, wherein the small plates, rods or segments (2) are connected to the distal part of the outer ( ) or inner (4) trocar sleeve, characterised in that
- the outer (3) and inner (4) sleeve move independently of each other and freely within each other, that
- the small plates, rods and segments (2) are independent parts which are connected in a hinge-like manner to the outer (3) or inner (4) trocar sleeve, and that
- adjusting the mover causes lateral swivelling by up to 90° of the small plates, rods or segments (2) at the distal end of the trocar sleeve, due to relative motion between the outer (3) and inner (4) trocar sleeve.

## Revendications

1. Manchon de trocart chirurgical comportant des éléments mobiles pivotants (1) et un dispositif de déplacement qui provoque le pivotement, l'écartement, l'ouverture d'au moins une lamelle, une tige ou un segment (2) reliés au dispositif, pour une utilisation en chirurgie, en particulier lors d'interventions chirurgicales endoscopiques peu étendues et intracorporelles, sous la forme d'un manchon de trocart multifonctionnel grâce auquel on peut introduire des endoscopes, des instruments et des éléments similaires à l'intérieur du corps, et repousser, tenir ou soulever des organes, des tissus ou la paroi abdominale,
- ledit manchon de trocart chirurgical se composant d'un manchon extérieur (3) et d'un manchon intérieur (4), étant précisé que les lamelles, tiges, segments (2) sont reliés à la partie distale des manchons extérieur (3) ou intérieur (4),
caractérisé
- en ce que les manchons extérieur (3) et intérieur (4) sont mobiles indépendamment l'un de l'autre et librement l'un dans l'autre,
- en ce que les lamelles, tiges, segments (2) sont des éléments indépendants qui sont reliés aux manchons extérieur (3) ou intérieur (4) à la manière de charnières,
- et en ce que pour le dispositif de déplacement, un déplacement relatif entre les manchons de trocarts extérieur (3) et intérieur (4) provoque un pivotement latéral jusqu'à 90° des lamelles, tiges, segments (2) au niveau de l'extrémité distale du manchon de trocart.
